# EUROPEAN PATENT APPLICATION

(11) **EP 2 386 641 A1**
(43) Date of publication of application: **16.11.2011**
(21) Application number: 10162603.4
(22) Date of filing: 11.05.2010
(51) Int. Cl.: C12N 15/67, C12N 15/73

(54) **Method for extracellular production of recombinant biomolecules in a microorganism**

(71) Applicant: Université Catholique de Louvain, 1348 Louvain-La-Neuve (BE)
(72) Inventor: Soumillion, Patrice, 1360 Perwez (BE); Baudoux, Bruno, 5000 Namur (BE)
(74) Representative: Icosa

(57) **Abstract**

The present invention relates to a method for producing into the extracellular medium a biomolecule of interest, the method comprising the steps of:
(a) culturing a recombinant microorganism co-expressing:
- the mutant pIV^{S324G} porine into the outer membrane of the microorganism and
- a recombinant biomolecule of interest into the periplasm of the microorganism,
thereby secreting the biomolecule of interest into the extracellular medium; and

(b) recovering the biomolecule of interest from the extracellular medium.

## Description

### FIELD OF THE INVENTION

The present invention relates to a method for producing and secreting into the extracellular medium a protein of interest, using a microorganism co-expressing a mutant pIV protein and the recombinant biomolecule of interest, said recombinant biomolecule being secreted into the extracellular medium.

### BACKGROUND OF THE INVENTION

Due to its simple genetic accessibility and competitive cultivation costs, Escherichia coli is and remains among the most frequently used hosts for recombinant protein production. High-level protein expression with products titers in multi-gram-per-liter range is achieved by the generation of optimized expression systems. In fact, nearly 40% of all recombinant therapeutic proteins are produced in Escherichia coli systems.

The major bottlenecks and manufacturing costs associated to production of a recombinant protein in Escherichia coli are nowadays the separation and purification of the recombinant protein, as Escherichia coli fails to produce the recombinant proteins into the extracellular medium. In fact, due to the absence of export machinery, proteins expressed in Escherichia coli accumulate into the cells. Consequently, the recombinant proteins suffer from increased degradation by cytoplasmic proteases or form aggregated inclusion bodies which lack biological activity. Complex and expensive denaturation and refolding procedures are then required to obtain biologically active proteins from the inclusion bodies.

A method for secreting recombinant biomolecules into the extracellular medium would thus be of real interest for the industry. In addition to prevent intracellular proteolysis and maybe also formation of inclusion bodies, the secretion of recombinant proteins into the extracellular medium presents the other advantage of having the recombinant protein maintained at high purity in the extracellular medium as little or no protein are secreted by Escherichia coli, thus allowing an easy isolation of the recombinant proteins.

Different methods were proposed to engineer Escherichia coli to produce extracellular proteins (Ni et al. Biotechnol Lett 2009, 31:1661-1670):
(a) engineering dedicated secretion systems that naturally exist in Escherichia coli pathogens;
(b) use of carrier proteins with no known translocation mechanisms;
(c) use of cell envelope mutants;
(d) co-expression of a lysis-promoting protein.

Patent application WO2010/012972 also suggested a method for increasing bacterial cell permeability properties by using the orf18 gene.

These methods however present several drawbacks. Type I secretion system mechanism is capable of exporting a protein of interest into the culture medium; however, the secreted peptide remains attached to its signal sequence and therefore an additional cleavage step is needed. Another drawback is that they may involve partial or total lysis of the microorganisms. Thus, amounts of intracellular protein from the microorganisms contaminate the extracellular medium and make it difficult to achieve an easy purification procedure.

Therefore, the present invention aims to provide an efficient and simple method for producing a recombinant biomolecule in the extracellular medium by expressing a mutant pIV protein into the outer membrane of the microorganisms.

The pIV protein exists as a homomultimer in the outer membrane of Escherichia coli and is specific of the export of the filamentous phage f1 (Marciano et al., Science 1999 284, 1516-1519) out of the bacteria. The pIV protein (called porine) is known to sequentially open in order to export phage f1 (and phage f1 only), and to close after exportation, until another opening/closing sequence. Marciano et al. described a mutant pIV porin: pIV^{S324G} allowing the diffusion of vancomycin into the periplasm of Escherichia coli and as well as sugars having a size up to maltohexahose within the bacteria.

However, the exact mechanism of the opening/closing of the native pIV protein or of the mutant pIV protein is still unknown, and for one skilled in the art, this protein may be considered as channel opening or closing in response to specific signals and not as an always-opened exporting channel, as if it was so, the content of the periplasm of the bacteria would leak out, and the bacteria could not survive.

Surprisingly, according to the invention, the inventors made it possible to export large proteins out of the periplasm of Escherichia coli through porin pIV^{S324G}, and make it a secretion tool.

The method of the invention thus allows an efficient secretion of recombinant protein from the periplasm to the extracellular medium, thereby providing an interesting tool for high yielding and cost-effective bioproduction of a soluble protein of interest.

### SUMMARY OF THE INVENTION

One object of the invention is a method for producing into the extracellular medium a biomolecule of interest, the method comprising the steps of:
(a) culturing a recombinant microorganism co-expressing:
   - the mutant pIV^{S324G} porine into the outer membrane of the microorganism and
   - a recombinant biomolecule of interest, wherein the recombinant biomolecule is expressed into the periplasm of the microorganism,
thereby secreting the biomolecule of interest into the extracellular medium; and (b) recovering the biomolecule of interest from the extracellular medium.

In one embodiment of the invention, the nucleic acid sequence encoding the mutant pIV^{S324G} porine is integrated into the chromosome of the microorganism.

In another embodiment of the invention, the nucleic acid sequence encoding the mutant pIV^{S324G} porine is comprised into an expression system that allows its expression into the outer membrane of the microorganism.

In one embodiment of the invention, the nucleic acid sequence encoding the biomolecule of interest is comprised into an expression system.

Another object of the invention is a method for producing into the extracellular medium a biomolecule of interest, the method comprising the steps of:
(a) culturing a recombinant microorganism co-transformed with:
   - a first expression system allowing the production of the mutant pIV^{S324G} porine into the outer membrane of the microorganism and
   - a second expression system allowing the production of the recombinant biomolecule of interest into the periplasm of the microorganism,
      thereby secreting the protein of interest into the extracellular medium; and
(b) recovering the recombinant biomolecule of interest from the extracellular medium.

In one embodiment of the invention, the recombinant microorganism is a gram-negative bacterium. In a preferred embodiment of the invention, the gram-negative bacterium is Escherichia coli.

In one embodiment of the invention, the first expression system comprises a low copy plasmid comprising the gene of the mutant porin under the control of a regulatable promoter. In a preferred embodiment of the invention, the low copy plasmid is the pPRO33 and the promoter is the PprpB or the Plac promoter.

In another embodiment of the invention, the second expression system is a high copy plasmid. In a preferred embodiment of the invention, the nucleic acid sequence of the biomolecule of interest is under the control of the T7 promoter or the P_{BAD} promoter.

In another embodiment of the invention, the second expression system comprises the nucleic acid sequence of the biomolecule of interest fused to a signal sequence for addressing the biomolecule of interest to the periplasm. In a preferred embodiment of the invention, the signal sequence is the DsbA sequence (MKKIWLALAGLVLAFSASA).

In one embodiment of the invention, at least 0,1 g/l of the protein of interest is recovered in the extracellular medium.

### DETAILED DESCRIPTION OF THE INVENTION

One object of the invention is a method for producing into the extracellular medium a biomolecule of interest, the method comprising the steps of:
(a) culturing a recombinant microorganism co-expressing:
   - the mutant pIV^{S324G} porine into the outer membrane of the microorganism and
   - a recombinant biomolecule of interest, wherein the recombinant biomolecule is expressed into the periplasm of the microorganism,
thereby secreting the biomolecule of interest into the extracellular medium; and (b) recovering the biomolecule of interest from the extracellular medium.

In one embodiment of the invention, the nucleic acid sequence encoding the mutant pIV^{S324G} porine is integrated into the chromosome of the microorganism.

In another embodiment of the invention, the nucleic acid sequence encoding the mutant pIV^{S324G} porine is comprised into an expression system that allows its expression into the outer membrane of the microorganism.

In one embodiment of the invention, the nucleic acid sequence encoding the biomolecule of interest is comprised into an expression system that allows its expression into the periplasm of the microorganism.

Another object of the invention is a method for producing into the extracellular medium a biomolecule of interest, the method comprising the steps of:
(a) culturing a recombinant microorganism co-transformed with:
   - a first expression system allowing the production of the mutant pIV^{S324G} porine into the outer membrane of the microorganism and
   - a second expression system allowing the production of the recombinant biomolecule of interest into the periplasm of the microorganism,
thereby secreting the protein of interest into the extracellular medium; and (b) recovering the recombinant biomolecule of interest from the extracellular medium.

As a result, when the mutant pIV^{S324G} porine and the biomolecule of interest are co-expressed, the biomolecule of interest can be efficiently secreted from the recombinant microorganism into the cell culture broth, and the secreted biomolecule of interest can be isolated and purified in a very simple manner as it is present into the cell culture broth.

### The mutant pIV^{S324G} porine

The mutant pIV^{S324G} porine was described in Marciano et al (Science 1999, 284: 1516-1519). The nucleic acid sequence of the mutant pIV^{S324G} porine is described in SEQ ID NO: 1 and its amino acids sequence in SEQ ID NO: 2.

As shown here after, the sequence of the signal peptide of the porine is italicized and the sequence of the mature porine starts at a glutamine (Q₁, first line). S324G mutation is highlighted in bold.

Atgaaattgttaaatgtaattaattttgttttcttgatgtttgtttcatcatcttcttttgctcaagta
M K L L N V I N F V F L M F V S S S S F A Q1 V
Attgaaatgaataattcgcctctgcgcgatttcgtgacttggtattcaaagcaaacaggtgaatctgtt
I E M N N S P L R D F V T W Y S K Q T G E S V
Attgtctcacctgatgttaaaggtacagtgactgtatattcctctgacgttaagcctgaaaatttacgc
I V S P D V K G T V T V Y S S D V K P E N L R
Aatttctttatctctgttttacgtgctaataattttgatatggttggctctaatccttccataattcag
N F F I S V L R A N N F D M V G S N P S I I Q
Aaatataacccaaatagtcaggattatattgatgaattgccatcatctgatattcaggaatatgatgat

K Y N P N S Q D Y I D E L P S S D I Q E Y D D
Aattccgctccttctggtggtttctttgttccgcaaaatgataatgttactcaaacatttaaaattaat
N S A P S G G F F V P Q N D N V T Q T F K I N
Aacgttcgcgcaaaggatttaataaggttgtagaattgtttgttaaatctaatacatctaaatcctca
N V R A K D L I R V V E L F V K S N T S K S S
Aatgtattatctgttgatggttctaacttattagtagttagcgcccctaaagatattttagataacctt
N V L S V D G S N L L V V S A P K D I L D N L
Ccgcaatttctttctactgttgatttgccaactgaccagatattgattgaaggattaatttcgaggtt
P Q F L S T V D L P T D Q I L I E G L I F E V
Cagcaaggtgatgctttagattttcctttgctgctggctctcagcgcggcactgttgctggtggtgtt
Q Q G D A L D F S F A A G S Q R G T V A G G V
Aatactgaccgtctaacctctgttttatcttctgcgggtggttcgttcggtatttttaacggcgatgtt
N T D R L T S V L S S A G G S F G I F N G D V
Ttagggctatcagttcgcgcattaaagactaatagccattcaaaaatattgtctgtgcctcgtattctt
L G L S V R A L K T N S H S K I L S V P R I L
Acgctttcaggtcagaagggttctatttctgttggccagaatgtcccttttattactggtcgtgtaact
T L S G Q K G S I S V G Q N V P F I T G R V T
Ggtgaatctgccaatgtaaataatccatttcagacaattgagcgtcaaaatgttggtatttctatgagt
G E S A N V N N P F Q T I E R Q N V G I S M S
Gtttttcccgttgcaatggctggcggtaatattgttttagatataaccagtaaggccgatagtttgggt
V F P V A M A G G N I V L D I T S K A D S L G
Tcttctactcaggcaagtgatgttattactaatcaaagaagtattgcgacaacggttaatttgcgtgat
S S T Q A S D V I T N Q R S I A T T V N L R D
Ggtcagactcttttgctcggtggcctcactgattacaaaaacacttctcaagattctggtgtgccgttc
G Q T L L L G G L T D Y K N T S Q D S G V P F
Ctgtctaaaatccctttaatcggcctcctgtttagctcccgttctgattctaacgaggaaagcacgttg
L S K I P L I G L L F S S R S D S N E E S T L
tacgtgctcgtcaaagcaaccatagtacgcgccctgtag
Y V L V K A T I V R A L Stop

### Expression systems

As used herein, "expression system" is a linear or a circular DNA molecule composed of a fragment encoding a target polypeptide operably linked to an additional fragment for the transcription of the system. The additional fragment includes a promoter and a stop codon sequence. The expression system contains one or more origins of replication, one or more selection markers, a sequence encoding a ribosome binding site. The expression system is generally originated from plasmid or virus DNA or contains elements from both.

"Operably linked" means that fragments are arranged to be functioning as they are supposed to be, for example once transcription starts at the promoter, it goes through coded fragment to stop codon.

Non-limiting examples of expression plasmids that can be used in the present invention are pET, pACYCs, pBAD, pTrc, pDUET, pPro, pGEX, pRSET, and pQE30.

In another embodiment of the invention, the linear or circular DNA construct can be integrated into the bacterial genome and may then be termed "expression cartridge" or "cartridge". As a result of integration, the expression host cell has an integrated "expression cassette". Preferably, the cartridge is a linear DNA construct comprising essentially a promoter, a gene of interest, a sequence encoding a ribosome binding site and two terminally flanking regions which are homologous to a genomic region and which enable homologous recombination. In addition, the cartridge may contain other sequences as described below in detail; e.g. sequences coding for antibiotic selection markers, prototrophic selection markers or fluorescent markers, markers coding for a metabolic gene, genes which improve protein expression (like e.g. the T7 RNA polymerase gene) or two flippase recognition target sites (FRT) which enable the removal of certain sequences (e.g. antibiotic resistance genes) after integration.

The cartridge is synthesized and amplified by methods known in the art, in the case of linear cartridges, usually by standard polymerase chain reaction, PCR (Wilson and Walker, principles and Techniques of Biochemistry and Molecular Biology, 2005, 6^{th} Ed., Cambridge University Press). Since linear cartridges are usually easier to construct, they are preferred for obtaining the expression host cells used in the method of the invention. Moreover, the use of a linear expression cartridge provides the advantage that the genomic integration site can be freely chosen by the respective design of the flanking homologous regions of the cartridge. Thereby, integration of the linear expression cartridge allows for greater variability with regard to the genomic region.

### Promoters

"Promoter" in the meaning of the present invention is an expression control element that permits binding of RNA polymerase and the initiation of transcription. In one embodiment of the invention, the gene of interest is under the control of a "strong" promoter. A strong promoter is characterized by a high binding affinity of the promoter sequence to an RNA polymerase, usually the naturally occurring corresponding RNA polymerase, on the one hand and the rate of formation of mRNA by that RNA polymerase on the other hand.

Preferably, the gene of interest is under the control of an inducible promoter. An inducible promoter is a promoter that is regulable by external factors, e.g. the presence of an inductor (also termed "inducer") molecule or the absence of a repressor molecule, or physical factors like increased or decreased temperature, osmolarity, or pH value. Different promoters and the respective induction principles were reviewed by Makrides et al. (Microbiological Reviews, 1996, (60)3: 512-538).

In the present invention, promoters may be used that have been developed for plasmid-based expression of the gene of interest, among the strong promoters, the T7 promoter of bacteriophage T7 has been most widely used.

In a preferred aspect, the promoter is the T7 promoter. T7-based expression systems, comprising a combination of the T7 promoter and T7 polymerase gene under the control of the lac promoter, are widely used for large scale expression of recombinant proteins in both bacterial and eukaryotic cells. The system makes use of the T7 RNA polymerase, transcription rate of which is several times higher than E. coli RNA polymerase. The expression from the T7 promoter is under the control of T7 RNA polymerase, which is stringently specific for the T7 promoter, i.e. the T7 promoter can only be utilized by the RNA polymerase of bacteriophage T7. When IPTG is added to the culture medium, T7 RNA polymerase is expressed by transcription from the lac promoter.

In the meaning of the present invention, the term "T7 promoter" includes promoters that are present in the genome of bacteriophage T7, as well as consensus sequences and variants of such promoters with the ability to mediate transcription by the T7 RNA polymerase. The bacteriophage T7 contains seventeen different promoter sequences, all of which comprise a highly conserved nucleotide sequence.

In another embodiment of the invention, the gene of interest or the mutant pIV^{S324G} porine may be under the control of the tac or the trc promoter, the lac or the lacUV5 promoter (all inducible by lactose or its analoge IPTG (isopropylthiol-β-D-galactoside)), the tightly regulatable araBAD promoter (P_{BAD}; Guzman et al., 1995, inducible by arabinose), the trp promoter (inducible by β-insole acrylic acid addition or tryptophan starvation, repressible by tryptophan addition), the lambda promoter pL (λ) (induction by an increase of temperature), the phoA promoter (inducible by phosphate starvation), the PprpB (induction with propionate) or other promoters suitable for recombinant protein expression, which all use E. coli RNA polymerase.

Among inducible promoters are those that show a "leaky" expression behavior. Such promoters (so-called "leaky promoters") are, in principle, inducible, but show nevertheless also basal expression without being externally induced. Inducible promoters that show leaky expression under non-induced conditions may behave similarly to constitutive promoters (i.e. they are steadily and continuously active or they may be activated or enhanced as a result of certain cultivation conditions). Leaky promoters may be particularly useful for continuously operated cultivation processes. Examples are the T7 promoter and the trp promoter. In the method of the invention, the promoter may also be constitutive, i.e. a promoter which controls expression without the need for induction on the one hand, or the possibility of repression on the other hand. Hence, there is continuous and steady expression at a certain level. Aside the advantage that they do not require an inducer, constitutive promoters are particularly useful in embodiments where the method is continuous (as described below). As an example, the strong constitutive HCD promoter (Poo et al., Biotechnology Letters, 2002, 24:1185-1189; Jeong et al., Protein expression and purification, 2004, 36:150-156) may be applied for constitutive expression.

In a preferred embodiment of the invention, promoters used for the expression of the gene of interest are strong promoters as for example T7 promoter or pBAD promoter.

In a preferred embodiment of the invention, promoters used for the expression of the mutant pIV^{S324G} porine are tunable and moderate promoters such as PprbB or the lac promoter. pPRO expression vectors using the PprpB promoter are described in Lee et al (Applied Microenvironmental Microbiology 2005, p6855-6882) and Lee et al (Protein Expr Purif, 2008, 61(2): 197-203).

### Selection Markers

Positive clones, i.e. clones that carry the expression vector or expression cassette, can be selected by means of a marker gene. In some embodiments, host cells are used that already contain a marker gene integrated in their genome, e.g. an antibiotic resistance gene or a gene encoding a fluorescent protein, e.g. GFP. In this case, the expression vector or expression cartridge which does not contain a selection marker, is integrated at the locus of the chromosomal marker gene, and positive clones are selected for loss/disappearance of the respective phenotype, e.g. they are selected for antibiotic sensitivity or disappearance of fluorescence, which can be directly visualized on the cultivation plates. These embodiments have the advantage that the marker is either interrupted or completely replaced by the expression cassette, and thus no functional marker sequence is present after integration and does not need to be removed, if undesirable, as in the case of antibiotic resistance genes.

The marker gene is part of the expression vector or expression cartridge. In the case that the marker used for selection is a gene conferring antibiotic resistance (e.g. for example kanamycin or chloramphenicol), positive clones are selected for antibiotic resistance (i.e. growth in the presence of the respective antibiotic).

The marker gene (irrespective of whether it is present on the host cell's genome or has been introduced by means of the expression vector or expression cartridge) can be eliminated upon integration of the cassette, e.g. by using the FLP recombinase function based on the site-specific recombination system of the yeast 2 micron plasmid, the FLP recombinase and its recombination target sites FRTs (Datsenko and Wanner, 2000). In certain embodiments, the expression cell has been engineered to carry a defective selectable marker gene, e.g. part of an antibiotic resistance gene like chloramphenicol or kanamycin, or part of a fluorescent marker or part of a gene involved in a metabolic pathway of a sugar or an amino acid. In this case, the cartridge or expression vector with the gene of interest carries the missing part of the marker gene, and by integration the marker gene restores its functionality.

In the case of an antibiotic resistance gene, the cells carrying the expression system are resistant against the specific antibiotic, in the case of a fluorescent marker cells can be visualized by fluorescence, and in the case of a metabolic pathway gene, cells obtain the ability to metabolize the respective component.

In certain embodiments, selection of positive clones (i.e. clones that carry the expression cassette or vector) is carried out by correction (i.e. complementation) of an auxotrophy of the host cell. In such embodiments, a host cell is used that has a mutation that has been chosen to allow selection of positive transformant colonies in an easy way, e.g. a strain that has a deletion or mutation that renders it unable to synthesize a compound that is essential for its growth (such mutation being termed as "auxotrophic marker"). For example, a bacterial mutant in which a gene of the proline synthesis pathway is inactivated, is a proline auxotroph. Such a strain is unable to synthesize proline and will therefore only be able to grow if proline can be taken up from the environment, as opposed to a proline prototroph which can grow in the absence of proline.

Any host cell having an auxotrophic marker may be used. Preferably, mutations in genes required for amino acid synthesis are used as auxotrophic markers, for instance mutations in genes relevant for the synthesis of proline, leucine or threonine, or for co-factors like thiamine. According to the invention, the auxotrophy of host cells is corrected by integration of the missing or defective gene as a component of the expression vector or expression cartridge into the genome along with integration of the gene of interest. The thus obtained prototrophic cells can be easily selected by growing them on a so-called "minimal medium" (prototrophic selection), which does not contain the compound for which the original host cell is auxotroph, thus allowing only positive clones to grow.

Prototrophic selection is independent of the integration locus. The integration locus for prototrophic selection may be any gene in the genome or at the locus carrying the auxotrophic marker. The particular advantage of prototrophic selection is that no antibiotic resistance marker nor any other marker that is foreign to the host remains in the genome after successful integration. Consequently, there is no need for removal of said marker genes, providing a fast and simple cloning and selection procedure. Another advantage is that restoring the gene function is beneficial to the cell and provides a higher stability of the system.

Alternatively, the marker gene that is inserted into the genome together with the expression system or expression cartridge, may be a metabolic gene that allows a particular selection mode. Such a metabolic gene may enable the cell to grow on particular (unusual) sugar or other carbon sources, and selection of positive clones can be achieved by growing cells on said sugar as the only carbon source.

In some embodiments (in the case that the protein of interest allows for detection on a single-cell or single-colony basis, e.g. by FACS analysis or immunologically (ELISA)), no marker gene is required, since positive clones can be determined by direct detection of the protein of interest.

### Signal sequences

One requirement for the expression vector system comprising the nucleic acid sequence of the biomolecule of interest is to also comprise at least one signal sequence that allows the expression of the polypeptide of interest into the periplasm.

Three pathways may be used for secretion across the bacterial cytoplasmic membrane: the SecB-dependent pathway, the signal recognition particle-dependent pathway (SRP), and the twin-arginine translocation (TAT) pathways (Mergulhao et al., 2005, Biotechnology Advances, 23:177-202).

Examples of suitable signal sequences to direct proteins to the periplasm of E. coli include the E. coli PhoA, PelB, SpA, Cex, DsbA, Exl, Enx, SthII, degQ, degS, degP, OmpA, OmpP, OmpT, LamB, OmpF and Bla (TEM-1 beta-lactamase) signal sequences (see Mergulhao et al., 2005, Biotechnology Advances, 23:177-202 for references).

The signal sequence may be linked to the N-terminal end of the polypeptide of interest or linked after some modification (Abrahmsen et al., EMBO J., 1985, 4:3901; Choi and Lee, Appl. Microbiol Technol., 2004, 64:625; Jobling et al., Plasmid, 1997, 38:158; Klein et al., Protein Eng. 1992, 5 :511; Ustumi et al., Gene, 1988, 71 :349).

In a preferred embodiment, the sequence signal is a sequence signal promoting co-translational export such as the DsbA sequence (MKKIWLALAGLVLAFSASA, SEQ ID NO: 3).

### Host cells

According to the invention, the microorganism host cells may be gram negative bacterial cells.

Preferably, the microorganism is Escherichia coli. In the method of the invention, any strain of E. coli may be used that can be cultivated in a defined medium.

Preferably, a strain derived from K-12 is used. The selection of an appropriate host strain may be based on the host's genotype, i.e. the specific mutations characterizing the strain, and/or on systematic comparison of several strains in experimental fermentations.

In a preferred embodiment of the invention, the strain has a mutation in the relA gene. This gene is responsible for the synthesis of guanosine tetraphosphate (ppGpp), a signal molecule which triggers the so-called "stringent response" in the cell upon amino acid limitation. In relA+ strains, amino acid limitation leads to a down-regulation of essential metabolic pathways involved in DNA replication and transcription. Strains having a mutation in the relA gene therefore continue to grow and to replicate plasmids upon amino acid limitation, which has been shown to be an important prerequisite for growing cells in a chemically defined medium with high plasmid replication rates.

In a further preferred embodiment of the invention, host strains having, in addition to a relA mutation, mutations in the genes of endA (endonuclease A) and of recA (recombination system) are used, since these mutations increase the structural integrity of the plasmid. Examples for suitable host strains are DH1, DH5, DH5-alpha, MC4100, TG1, TG2, DHB4, DH10B, JM108, JM109, BL21, XL1Blue and HB101.

Preferably, the host cell has the property to allow cultivation to high cell densities.

Another possible requirement to the host cell is that it contains an RNA polymerase that can bind to the promoter controlling the gene of interest. The RNA polymerase may be endogenous or foreign to the host cell. Preferably, host cells with a foreign strong RNA polymerase are used. Most preferably host strains, e.g. E. coli strains, are used that have been engineered to carry a foreign RNA polymerase (like e.g. in the case of using a T7 promoter a T7-like RNA polymerase in the so-called "T7 strains") integrated in their genome.

Examples for T7 strains are widely used and commercially available, e.g. BL21(DE3), HMS174(DE3) and their derivatives or relatives (Novagen, pET System manual, 11th edition). These strains are DE3 lysogens containing the T7 RNA polymerase gene under control of the lacUV5 promoter. Induction with IPTG allows production of T7 RNA polymerase which then directs the expression of the gene of interest under the control of the T7 promoter. Preferably, a host cell is used that contains a T7 RNA polymerase which has been genome - integrated without generating phage lysogens, e.g. as described in WO 2003/050240. (Cells that do not generate phage lysogens are "non-lysogenic").

A "T7-like RNA polymerase" includes, but is not limited to, RNA polymerases from other T7-like phages, such as the T3 RNA polymerase, as disclosed in US 4,952,496. A prerequisite of a T7-like RNA polymerase is that there should be a highly specific cognate promoter, and that the gene of interest is transcribed at a high rate in the presence of said RNA polymerase.

### Biomolecule of interest

The nucleic acid sequence encoding the biomolecule of interest fused to its signal sequence is also termed gene of interest.

With regard to the biomolecule of interest, there are no limitations. It may, in principal, be any polypeptide, protein or peptide that is to be produced on a manufacturing scale, e.g. an industrial biomolecule or a therapeutic biomolecule.

Examples for biomolecules that can be produced by the method of the invention are, without limitation, enzymes, regulatory proteins, receptors, peptides, e.g. peptide hormones, cytokines, membrane or transport proteins.

The biomolecules of interest may also be antigens as used for vaccination, vaccines, antigen-binding proteins, immune stimulatory proteins, allergens, full-length antibodies or antibody framents or derivatives. Antibody derivatives may be selected from the group of single chain antibodies, (scFv), Fab fragments, Fv fragments, single domain antibodies (V_{H} or V_{L} fragment), domain antibodies like camelid single domain antibodies (V_{HH}, nanobodies) or other antibody formats as described for instance in Andersen and Reilly (Current Opinion in Biotechnology, 2004, 15:456-462) or Holliger and Hudson (Nature Biotechnology, 2005 (23)9: 1126-1136). The expression of functional antibody fragments in E.coli is accomplished through secretion the light chain and heavy chain fragments into the periplasm separately, where proper assembly and disulfide bond formation can take place. Bivalent F(ab')2 fragment are necessary for clinical applications, which can provide longer circulating half time in patients than Fab' fragment does. Antibody fragments are prone to be degraded and form inclusion bodies when expressed in periplasm. Secreting the antibody fragment further into medium once it is translocated into periplasm greatly diminish the above problems.

The biomolecules of interest in the present invention can be exemplified by protein (viral antigen), e.g., coat protein, core protein, protease, reverse transcriptase, integrase, and so forth, encoded in the genome of a pathogenic virus, e.g., hepatitis B virus, hepatitis C virus, I-HV, influenza, and so forth; growth factors such as platelet-derived growth factor (PDGF), stem cell growth factor (SCF), hepatocyte growth factor (HGF), transforming growth factor (TGF), nerve growth factor (NGF), epidermal growth factor (EGF), fibroblast growth factor (FGF), insulin-like growth factor (IGF), and so forth; cytokines such as tumor necrosis factor, interferon, interleukin, and so forth; hematopoietic factors such as erythropoietin, granulocyte colony-stimulating factor, granulocyte-macrophage colony-stimulating factor, macrophage colony-stimulating factor, thrombopoietin, and so forth; peptide hormones such as luteinizing hormone-releasing hormone (LB-RH), thyrotropin-releasing hormone (TRH), insulin, somatostatin, growth hormone, prolactin, adrenocorticotropic hormone (ACTH), melanocyte-stimulating hormone (MSH), thyroid-stimulating hormone (TSH), luteinizing hormone (LU), follicle-stimulating hormone (FSH), vasopressin, oxytoxin, calcitonin, parathyroid hormone (PTH), glucagon, gastrin, secretin, pancreozymin, cholecystokinin, angiotensin, human placenta lacto8en, human chorionic gonadotropin (HCG), cerulein, motilin, and so forth; analgesic peptides such as enkephalin, endorphin, dynorphin, kyotorphin, and so forth; enzymes such as superoxide dismutase (SOD), urokinase, tissue plasminogen activator (TPA), asparaginase, kallikrein, and so forth; peptide neurotransmitters such as bombesin, neutrotensin, bradykinin, substance P, Alzheimer's amyloid peptide (AD), SODl, presenillin 1 and 2, renin, D-synuclein, amyloid A, amyloid P, activin, anti-HER-2, bombesin, enkephalinase, protease inhibitors, therapeutic enzymes, D l-antitrypsin, mammalian trypsin inhibitor, mammalian pancreatic trypsin inhibitor, calcitonin, cardiac hypertrophy factor, cardiotrophins (such as cardiotrophin-1), CD proteins (such as CD-3, CD-4, CD-8 and CD-19), CFTR, CTNF, DNase, human chorionic gonadotropin, mouse gonadotropin-associated peptide, cytokines, transthyretin, amylin, lipoproteins, lymphokines, lysozyme, a growth hormone (including human growth hormone), bovine growth hormone, growth hormone releasing factor, parathyroid hormone, thyroid stimulating hormone, growth factors, brain-derived neurotrophic growth factor, epidermal growth factor (EGF), fibroblast growth factor (such as D FGF and D FGF), insulin-like growth factor-I and -II, des(l-3)-IGF-I (brain IGF-I), insulin-like growth factor binding proteins, nerve growth factor (such as NGF- D), platelet-derived growth factor (PDGF), vascular endothelial growth factor (VEGF), receptors for growth hormones or growth factors, transforming growth factor (TGF) (such as TGF-D, TGF-D 1, TGF-D2, TGF-D3, TGF-D4 or TGF-D5), neurotrophic factors (such as neurotrophin-3, -4 ,-5, or -6), gelsolin, glucagon, kallikreins, mullerian- inhibiting substance, neurotrophic factors, p53, protein A or D, prorelaxin, relaxin A-chain, relaxin B-chain, rheumatoid factors, rhodopsin, a serum albumin (such as human serum albumin), inhibin, insulin, insulin chains, insulin A-chain, insulin D -chain, insulin receptor, proinsulin, luteinizing hormone, integrin, interleukins (ILs) (such as IL-1 to IL-10, IL-12, IL-13), erythropoietin, thrombopoietin, fibrillin, follicle stimulating hormone, clotting factors (such as factor VIIIC, factor IX, tissue factor, and von Willebrands factor, anti-clotting factors (such as Protein C, atrial naturietic factor, lung surfactant), a plasminogen activator (such as human tissue plasminogen activator or urokinase), thrombin, tumor necrosis factor- D or D, D-ketoacid dehydrogenase, addressins, bone morphogenetic proteins (BMPs), collagen, colony stimulating factors (CSFs) (such as M-CSF, GM-CSF and G-CSF), decay accelerating factor, homing receptors, interferons (such as interferon-alpha , -gamma and -beta), keratin, osteoinductive factors, PRNP, regulatory proteins, superoxide dismutase, surface membrane proteins, transport proteins, T- cell receptors, antigens such as gpl 20(HIb) immuno toxins, atrial natriuretic peptide, seminal vesicle exocrine protein, D 2- microglobulin, PrP, precalcitonin, ataxin 1, ataxin 2, ataxin 3, ataxin 6, ataxin 7, huntingtin, androgen receptor, CREB-binding protein, gpl 20, p300, CREB, API, ras, NFAT, jun, fos, dentaorubral pallidoluysian atrophy-associated protein, a microbial protein (e.g., maltose binding protein, ABC transporter, glutathione S transferase, thioredoxin, D -lactamase), green fluorescent protein, red fluorescent protein, an enzyme such as superoxide dismu-tase, asparaginase, arginase, arginine deaminase, adenosine deaminase, ribonuclease, catalase, uricase, bilirubin oxidase, trypsin, papain, alkaline phosphatase, beta-glucoronidase, purine nucleoside phosphorylase or batroxobin, an opioid, e.g. endorphins, enkephalins or non-natural opioids, a hormone or neuropeptide, e.g. calcitonin, glucagon, gastrins, adreno- corticotropic hormone (ACTH), cholecystokinins, lutenizing hormone, gonadotropin- releassing hormone, chorionic gonadotropin, corticotrophin-releasing factor, vasopressin, oxytocin, antidiuretic hormones, thyroid-stimulating hormone, thyrotropin-releasing hormone, relaxin, prolactin, peptide YY, neuropeptide Y, pancreastic polypeptide, leptin, CART (cocaine and amphetamine regulated transcript), a CART related peptide, perilipin, melano- cortins (melanocyte-stimulating hormones) such as MC-4, melanin-concentrating hormones, natriuretic peptides, adrenomedullin, endothelin, secretin, amylin, vasoactive intestinal peptide (VIP), pituary adenylate cyclase activating polypeptide (PACAP), bombesin, bombesin- like peptides, thymosin, heparin-binding protein, soluble CD4, hypothalmic releasing facto- rand melanotonins or functional analogs thereof. In another embodiment of the invention the target protein may be a processing enzyme such as proteases (eg enterokinase, caspases trypsine like serine proteases), lipase, phospatase, glycosyl hydrolases (eg. mannosidases, xylosidases, fucosidases), kinase, mono or dioxidase, peroxidase, transglutaminase, carboxypeptidase, amidase, esterase, and phosphatase...

Preferred sources for such mammalian polypeptides include human, bovine, equine, porcine, lupine and rodent sources, with human proteins being particularly preferred.

The biomolecule of interest of the present invention also encompasses variants of the aforementioned protein. These variants encompass, for example, protein that has the same activity as the aforementioned protein and that comprises an amino acid sequence with, in the amino acid sequence of the aforementioned protein, one or more deleted, substituted, inserted and/or added amino acids. Such protein can be exemplified by protein that has the same activity as the aforementioned protein and that comprises an amino acid sequence with, in the amino acid sequence of the aforementioned protein, one or more deleted, substituted, inserted and/or added amino acids. Two or more different types of modifications selected from deletion, substitution, insertion, and addition may be carried out concurrently.

The biomolecule of interest of the present invention also encompasses "partial peptides" of the aforementioned protein. A partial peptide of the protein can be exemplified by a partial peptide comprising an amino acid sequence in which a portion of the amino acid sequence of the aforementioned protein runs uninterrupted, wherein the partial peptide preferably has the same activity as said protein. Such a partial peptide can be exemplified by a polypeptide that has an amino acid sequence comprising at least 20 and preferably at least 50 of the amino acid residues in the amino acid sequence of the aforementioned protein. This polypeptide preferably contains the amino acid sequence that corresponds to the region that is involved with the activity of the aforementioned protein. In addition, the partial peptide used in the present invention may also be a partial peptide as yielded by a modification of this polypeptide wherein 1 or a plurality of amino acid residues (for example, approximately 1 to 20, more preferably approximately 1 to 10, and even more preferably approximately 1 to 5) is deleted from, substituted in, inserted into, and/or added to its amino acid sequence. The partial peptide used in the present invention can also be used as an antigen for antibody production.

In one embodiment of the invention, the biomolecule of interest is selected from the group comprising Human growth hormone, human insulin, follicle-stimulating hormone, Factor VIII, Erythropoeietin, Granulocyte colony-stimulating factor, Alpha-glactosidase A, Alpha-L-iduronidase, N-actetylgalactosamine-4-sulfatase, Dornase alfa, Tisssue plasminogen activator, Glucocerebrosidase, Interferon, Insulin-like growth factor 1, bovine somatotropin, Porcine somatotropin, bovine chymosin, and envelop protein of the hepaptitis B virus.

The biomolecule of interest also encompasses modified polypeptides or proteins that have underwent posttranslational and post-export modifications in the periplasm such as cyclization, glycosylation, phophorylation, methylation, oxidation, dehydratation, proteolytic cleavage.

As described here above, the inventors surprisingly found that the mutant pIV^{S324G} porine was capable to export from the host cell the expressed biomolecule of interest and in particular was capable to export from the host cell large biomolecule having a molar mass superior to 1500 g/mol).

According to the invention, the biomolecules of interest may be of any size. As shown in the Examples, the biomolecule of interest may be a small cyclic peptide or may be a larger protein such as tem-1 beta-lactamase (30 000 g/mol).

In one embodiment of the invention, the biomolecule of interest has a molar mass superior to 1500 g/mol.

In one embodiment of the invention, the biomolecule of interest has a molar mass superior to 1500 g/mol and inferior to 100000 g/mol, preferably 50000 g/mol.

According to another embodiment, the biomolecule of interest has a minimum dimension of 7nm or less, preferably of 0.5 to 6 nm, more preferably of 1 to 5 nm.

In a preferred embodiment, the biomolecule of interest has a molar mass superior to 5000 g/mol, preferably 15000 g/mol, more preferably 30000 g/mol and a minimum dimension of less than 6 nm.

### Manufacturing Scale Production

According to the invention, the term "cultivating" (or "cultivation", also termed "fermentation") relates to the propagation of bacterial expression cells in a controlled bioreactor according to methods known in the industry.

Manufacturing of recombinant proteins is typically accomplished by performing cultivation in larger volumes. The term "manufacturing" and "manufacturing scale" in the meaning of the invention defines a fermentation with a minimum volume of 5 L culture broth. Usually, a "manufacturing scale" process is defined by being capable of processing large volumes of a preparation containing the recombinant protein of interest, and yielding amounts of the protein of interest that meet, e.g. in the case of a therapeutic protein, the demands for clinical trials as well as for market supply. In addition to the large volume, a manufacturing scale method, as opposed to simple lab scale methods like shake flask cultivation, is characterized by the use of the technical system of a bioreactor (fermenter) which is equipped with devices for agitation, aeration, nutrient feeding, monitoring and control of process parameters (pH, temperature, dissolved oxygen tension, back pressure, etc.).

According to the invention, the method of the invention as described here above is intended for obtaining at least 0,1 g/l of the protein of interest, preferably at least 0,25 g/l, 0,5 g/l, 0,75 g/l, and most preferably at least 1 g/l, 2 g/l, 3 g/l, 4 g/l, 5 g/l of the protein of interest in the extracellular medium.

### Fed-batch Cultivation

In certain embodiments, the method of the invention is a fed-batch process.

Whereas a batch process is a cultivation mode in which all the nutrients necessary for cultivation of the cells are contained in the initial culture medium, without additional supply of further nutrients during fermentation, in a fed-batch process, after a batch phase, a feeding phase takes place in which one or more nutrients are supplied to the culture by feeding. The purpose of nutrient feeding is to increase the amount of biomass (so-called "High-cell-density-cultivation process" or "HCDC") in order to increase the amount of recombinant protein as well. Although in most cultivation processes the mode of feeding is critical and important, the present invention is not restricted with regard to a certain mode of feeding. Feeding of nutrients may be done in a continuous or discontinuous mode according to methods known in the art. The feeding mode may be pre-defined (i.e. the feed is added independently from actual process parameters), e.g. linear constant, linear increasing, stepwise increasing or following a mathematical function, e.g. exponential feeding.

In a preferred embodiment, the method of the invention is a fed-batch process, wherein the feeding mode is predefined according to an exponential function. By applying an exponential feeding mode, the specific growth rate µ of the cell population can be pre-defined at a constant level and optimized with respect to maximum recombinant protein expression. Control of the feeding rate is based on a desired specific growth rate µ. When a defined medium, as described below, is used, growth can be exactly predicted and pre-defined by the calculation of a biomass aliquot to be formed based on the substrate unit provided.

In another embodiment, an exponential feeding mode may be followed, in the final stages of cultivation, by linear constant feeding.

In another embodiment of the fed-batch process, linear constant feeding is applied. Linear constant feeding is characterized by the feeding rate (volume of feed medium per time unit) that is constant (i.e. unchanged) throughout certain cultivation phases.

In another embodiment of the fed-batch process, linear increasing feeding is applied. Linear increasing feeding is characterized by a feeding rate of feed medium following a linear function. Feeding according to a linear increasing function is characterized by a defined increase of feeding rate per a defined time increment.

In another embodiment of the fed-batch process of the invention, a feedback control algorithm is applied for feeding (as opposed to a pre-defined feeding mode). In a feedback-controlled fed-batch process, the feeding rate depends on the actual level of a certain cultivation parameter. Cultivation parameters suitable for feedback-controlled feeding are for instance biomass (and chemical or physical parameters derived thereof), dissolved oxygen, respiratory coefficient, pH, or temperature). Another example for a feedback controlled feeding mode is based on the actual glucose concentration in the bioreactor.

### Continuous and Semi-Continuous Cultivation Mode

In another embodiment, bacterial cells are cultivated in continuous mode (chemostat). A continuous fermentation process is characterized by a defined, constant and continuous rate of feeding of fresh culture medium into the bioreactor, whereby culture broth is at the same time removed from the bioreactor at the same defined, constant and continuous removal rate. By keeping culture medium, feeding rate and removal rate at the same constant level, the cultivation parameters and conditions in the bioreactor remain constant (so-called "steady state"). The specific growth rate µ can be pre-defined and is exclusively a result of the feeding rate and the culture medium volume in the bioreactor.

One advantage of cells having one or more genome-based expression cassettes is that they are genetically very stable (as opposed to structurally and segregationally instable plasmid-based expression systems, or expression systems which genome-inserted cassette relies on genomic amplification), the number of generations (cell doublings) of cells according to the invention is theoretically unlimited, as well as, consequently, cultivation time. The advantage of cultivating a genetically stable genome-based expression system in a continuous mode is that a higher total amount of recombinant protein per time period can be obtained, as compared to genetically unstable prior art systems. In addition, due to the theoretically unlimited time of cultivation, continuous cultivation of cells according to the invention may lead to a higher total protein amount per time period even compared to fed-batch cultivation processes.

Another embodiment of the invention refers to semi-continuous cultivation of cells. A semi-continuous cultivation process in the meaning of the invention is a process which is operated in its first phase as a fed-batch process (i. e. a batch phase followed by a feeding phase). After a certain volume or biomass has been obtained (i.e. usually when the upper limit of fermenter volume is obtained), a significant part of cell broth containing the recombinant protein of interest is removed from the bioreactor. Subsequently, feeding is initiated again until the biomass or volume of culture broth has again reached a certain value. This method (draining of culture broth and re-filling by feeding) can be proceeded at least once, and theoretically indefinite times.

### Culture Medium

With regard to the type of the culture medium used in the fermentation process, there are no limitations. The culture medium may be semi-defined, i.e. containing complex media compounds (e.g. yeast extract, soy peptone, casamino acids), or it may be chemically defined, without any complex compounds.

In the methods of the invention, significantly higher yields are obtained, because growth of bacteria and a high, but physiologically tolerable, recombinant gene expression rate can be maintained during the whole production process.

### Induction Mode

As described above, in a most preferred embodiment of the invention, the protein of interest is under control of an "inducible" or "controllable" promoter.

There is no limitation as regards the mode by which induction of protein expression is performed. By way of example, the inductor can be added as a singular or multiple bolus or by continuous feeding, the latter being also known as "inductor feed(ing)". There are no limitations as regards the time point at which the induction takes place. The inductor may be added at the beginning of the cultivation or at the point of starting continuous nutrient feeding or after (beyond) the start of feeding. Inductor feeding may be accomplished by either having the inductor contained in the culture medium or by separately feeding it.

The advantage of inductor feeding is that it allows controlling inductor dosage, i.e. it allows maintaining the dosage of a defined or constant amount of inductor per constant number of genes of interest in the production system. For instance, inductor feeding allows an inductor dosage which is proportional to the biomass, resulting in a constant ratio of inductor to biomass. Biomass units on which the inductor dosage can be based, may be for instance cell dry weight (CDW), wet cell weight (WCW), optical density, total cell number (TCN; cells per volume) or colony forming units (CFU per volume) or on-line monitored signals which are proportional to the biomass (e.g. fluorescence, turbidity, dielectric capacity, etc.). Essentially, the method of the invention allows the precise dosage of inductor per any parameter or signal which is proportional to biomass, irrespective of whether the signal is measured offline or on-line. Since the number of genes of interest is defined and constant per biomass unit (one or more genes per cell), the consequence of this induction mode is a constant dosage of inductor per gene of interest. As a further advantage, the exact and optimum dosage of the amount of inductor relative to the amount of biomass can be experimentally determined and optimized.

It may not be necessary to determine the actual biomass level by analytical methods. For instance, it may be sufficient to add the inductor in an amount that is based on previous cultivations (historical biomass data). In another embodiment, it may be preferable to add the amount of inductor per one biomass unit as theoretically calculated or predicted. For instance, it is well known for feeding- based cultivations (like fed-batch or continuous) that one unit of the growth- limiting component in the feed medium, usually the carbon source, will result in a certain amount of biomass. Consequently, a defined dosage of inductor per gene of interest may also be achieved by the defined dosage of inductor per unit growth limiting-component, since a certain unit of growth limiting component results in a defined unit of biomass, and a defined unit of biomass contains a defined number of molecules of proteins of interest according to the method of the invention.

As an essential advantage, feeding limiting amounts of inductor prevents metabolic load and reduces stress in favour of maximizing the capacity of protein synthesis.

The ratio of inductor per biomass (or per gene or per unit growth- limiting substrate) may not necessarily be constant. It may also be linear increasing, linear decreasing, increasing or decreasing according to exponential or other mathematical functions, etc.

In certain embodiments, the method of the invention is a fed-batch process, wherein the inductor is present in the batch medium from start of cultivation.

The mode of induction of expression can also be constitutive, which means that induction is not triggered chemically or by other stimuli, but that it is permanent from start of cultivation. In the absence of any toxicity, constitutive induction is the preferred induction mode for continuous cultivation, but also useful for fed-batch cultivation.

### BRIEF DESCRIPTION OF THE FIGURES

**Figure 1****:** Expression vector for the cyclic hexapeptide p6-4.
**Figure 2** **:** Schematic view of the secretion of a biomolecule of interest in E. coli expressing the mutant pIV porine.
**Figure 3****.** Growth curves of E.coli TOP10 strain transformed with pPRO30-pIV^{S324G} vector in LB medium and under various expression conditions.
**Figure 4** **:** Secretion of a cyclic peptide in the extracellular medium. MS analysis of extracellular and periplasmic extracts of *E.coli* TOP10 strain expressing the P6-4 peptide (cyclic SERELG, M+H+ = 672.32) with (bottom) or without (top) the coexpression of pIV^{S324G} pore protein. The periplasmic crude extracts were analyzed without any additional purification whereas, because of the high salts concentration in the culture medium, the extracellular extracts had to be desalted using a C₁₈ "ZipTip" column prior to analysis.
**Figure 5****:** Beta-lactamase activity in the extracellular medium. Light grey bars: Top10 expressing pIV^{S324G} and the tem-1 beta-lactamase. Dark grey bars: Top10 expressing only the tem-1 beta-lactamase.

### EXAMPLES

### Material and Methods

*Oligonucleotides.* Restriction sites are underlined.
*(SEQ ID NO: 4) -* IN-*fp : 5'-AAC AGT GGG TCT TCG TGA AGA CGC GGA TGC ATC AGT GGA GAT AG-3'*
*(SEQ ID NO: 5) - IN-rp : 5'-ATC TAG ACT ATA ATT GTA AAG AGG AGC T-3'*
*(SEQ ID NO: 6) - IC-fp: 5'-GCC CAT GGG ATC ACC AGA AAT AGA AAA G-3'*
*(SEQ ID NO: 7) - IC-rp : 5'-GCA TCC GCG TCT TCA CGA AGA CCC ACT GTT ATG GAC AA T GAT GTC-3'*
*(SEQ ID NO: 8) - INL₍₄₋₆₎-fp: 5'-G GGG GAA GAC TCC AGT (NNB)₄₋₆ GGA TGC ATC AGT GGA G-3'*
*(SEQ ID NO: 9) - INL-rp : 5'-GCC AAG CTG GAG ACC GTT TAA CT -3'*
*(SEQ ID NO: 10) - ICL-fp : 5'- CCC CGC TTA TTA AAA GCA TTC-3'*
*(SEQ ID NO: 11) - ICL-rp : 5'-CAG CAT TAC CCA GTC ACC ATG AAG ACC CAC TGT TAT GG -3'*

(SEQ ID NO: 12) - pIV-fp : 5'-ATT CAA CTG CTA GCA GGA GGA ATT CCA TAT GAA ATT GTT AAA
TGT AAT TAA TTT TG-3'

*(SEQ ID NO: 13) - pIV-rp : 5'-CTA ATC AAG GAT CCC TAC AGG GCG CGT ACT ATG G-3'*
*(SEQ ID NO: 14) - S324G-fp : 5'-GGC CGA TAG TTT GGG TTC TTC TAC TCA G-3'*
*(SEQ ID NO: 15) - S324G-rp : 5'-CTG AGT AGA AGA ACC CAA ACT ATC GGC C-3'*

### Plasmids constructions.

The fd-dog pIV gene was amplified and the mutation S324G simultanously added by overlap PCR. The two pairs of primers used were pIV-fp/S324G-rp and pIV-rp/S324G-fp. The primers pIV-fp and pIV-rp contain respectively a NheI and a BamHI restriction sites used to clone the PCR product within the vector pPRO30⁽⁴⁾ or pPRO33⁽⁴⁾. These plasmids are sold by Addgene. The primer pIV-fp codes as well for the Shine-Dalgarno sequence. The construct was confirmed by sequencing.

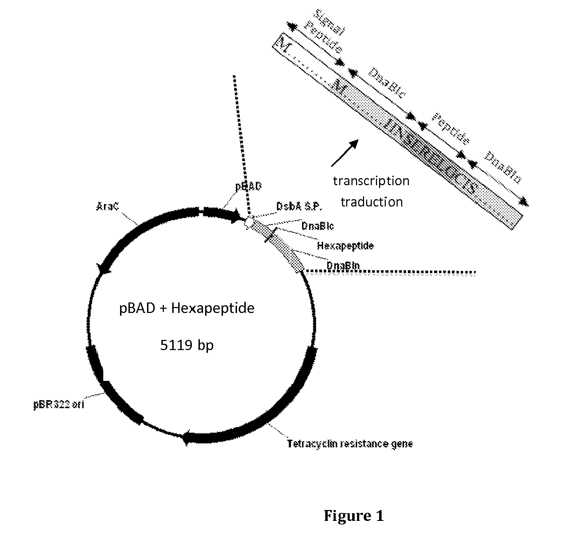

The vector for the expression of the P6-4 cyclic peptide was isolated form a collection of clones expressing randomized cyclic peptides that was build by Geneviève Deschuyteneer ⁽³⁾. For the library construction, the Ssp DnaBI_{N} and DnaBIc gene fragments were amplified from the cyanobacterium Synechocystis sp. strain PCC6803 (Laboratory of O. Vallon) with IN-fp/IN-rp and IC-fp/IC-rp primer pairs. The two PCR fragments were assembled by overlap extension. The final PCR product was cloned between the *NcoI* and *XbaI* sites into the pBAD vector, a derivative of pBAD/myc-HisB (Invitrogen) but carrying a tetracycline resistance gene instead of ampicillin. The C50A mutation in the DnaBI_{N} gene was introduced by Quick-Change mutagenesis (Stratagene), resulting in the plasmid pBICA. For the libraries, the DnaBI_{N} and DnaBIc gene fragments were amplified from the pBICA vector with INL-rp/INL₍₄₋₆₎-fp and ICL-fp/ICL-rp pairs of primers The two fragments were then restricted by BbsI, ligated together and finally cloned between the *NcoI* and *XbaI* site into the pBAD vector containing the DsbA signal sequence. Clones picked at random in the library were analysed for the periplasmic expression of cyclic peptides and the clone expressing the P6-4 peptide was chosen because of the high yield of peptide production and its easy detection by mass spectrometry analysis. A schematic representation of the expression vector is shown in **Figure 1****.**

The tem-1 beta-lactamase gene fused to his natural peptide signal was cloned in the same derivative of pBAD/myc-HisB vector described above. The construct is under control of the pBAD promoter. The amino acid sequence encoding the beta-lactamase is shown in SEQ ID NO: 16 and the amino acid sequence encoding its signal sequence is MSIQHFRVALIPFFAAFCLPVFA (SEQ ID NO: 17).

### Peptides expression.

*E.coli* Top 10 cells (Invitrogen) harboring vectors were grown O.N. at 37°C under shaking (180rpm) in 10ml of CAS medium⁽⁶⁾ (M9 minimal medium supplemented with casamino acids (12 g/l), tetracycline (7.5 µg/ml), L-tryptophan (2 µg/ml), CaCl₂ (0.1 mM) and MgCl₂ (1 mM) supplemented with ampicillin (100ug/ml) and glucose 1%. The cultures were centrifuged at 4500g for 5 minutes and the supernatant discarded. The pellet was resuspended in 0,5ml of CAS medium supplemented with ampicillin (100ug/ml) and propionate (0,5mM final). Once reached an O.D. of 0,6 at 600nm, the cultures were induced with L-(+)-arabinose to a final concentration of 0.2% and grown at 37°C for 3 h before doing a periplasmic extract. The periplasmic extracts and the supernatants were concentrated 5 times using a SpeedVac before MS analysis.

### Preparation of extracellular and periplasmic extracts.

Cultures were centrifuged at 4,400 x g for 10 min, and supernatants were recovered as extracellular extracts. The harvested cells were resuspended in 33 mL sucrose 20%, tris-HCl 20 mM pH 8. After 10 min incubation time at room temperature, cells were centrifuged 10 min at 6000 rpm. Supernatants were discarded and the osmotic shock was accomplished by resuspending the pellets in 50 mL MgSO₄ 5 mM. Samples was then incubated at 4°C for 10 min. Supernatants obtained after centrifugation at 10,000 x g for 10 min were recovered as periplasmic extracts.

### MS and MS/MS analysis.

Extracellular or periplasmic extracts were either treated as such or initially desalted using a MilliporeC18 ZipTip® (Bedford, MA, USA) after acidification with 1 µl of 10 % (v/v) TFA. 0.5 µl of each sample was added to 0,5 µl of a solution containing 10 mg/ml of alpha-cyano-4-hydroxycinnamic acid in 50 % (v/v) acetonitrile and 0.1 % (v/v) TFA. 0.5 µl of this mixture was put down on the target. MS and MS/MS spectra were acquired using an Applied Biosystems 4800 MALDI TOF/TOF™ Analyzer spectrometer using a 200 Hz solid state laser operating at 355 nm. MS spectra were obtained using a laser intensity of 3200 and 2000 laser shots by spot in a range of m/z between 500 and 4000. MS/MS spectra were obtained by selecting precursor ions corresponding to the precursor masses detected on the MS spectra in using a laser intensity of 3800 and 3000 laser shots by precursor. The automatically selected precursor fragmentation was performed at a collision energy of 1 kV without or with collision gas air at a pressure of about 1 x 10⁶ Torr. Data were collected with the Applied Biosystems 4000 Series Explorer™ software and MSMS spectra were analyzed with the Applied Biosystems GPS Explorer TM software - De Novo Explorer Version 3.6.

### Beta-lactamase exportation to the extracellular medium.

E.coli Top 10 cells (Invitrogen) harboring vectors (pPRO33/pIV^{S238G} or pPRO30 and pBAD/tem-1) were grown in 100ml of Luria-Bertani broth medium supplemented with tetracycline (7.5 µg/ml), chloramphenicol (34ug/ml) and propionate (0,5mM final) at 37°C until the culture reached an OD₆₀₀ of 0.6. The cultures were induced with L-(+)-arabinose to a final concentration of 0.2% and grown at 37°C for 4 h with an agitation of 180rpm. Every 30 minutes, 1ml of culture was sampled and centrifuged 1 minute at full speed using a table centrifuge.

The beta-lactamase activity of the supernatant was measured following the hydrolysis of nitrocefin at 482nm. In practice, we added to a photospectrometry cuvette 100ul of nitrocefin 0,3mM in phosphate buffer 100mM pH 7,5, 10 to 500ul of supernatant depending on the time elapsed after the induction and the volume was adjusted to 1ml with phosphate buffer 100mM pH 7,5.

### Beta-lactamase concentration estimation.

To estimate the beta-lactamase concentration in the medium after 4h of induction, we followed the hydrolysis of the penicillin G at 232nM to obtain the Vmax. Knowing the Kcat (1000 s⁻¹) it was possible to estimate the enzyme concentration. In practice, we added to a quartz cuvette 2ml of penicillin G 0,5mM in phosphate buffer 100mM pH7,5 and 10ul of the supernatant.

### Results

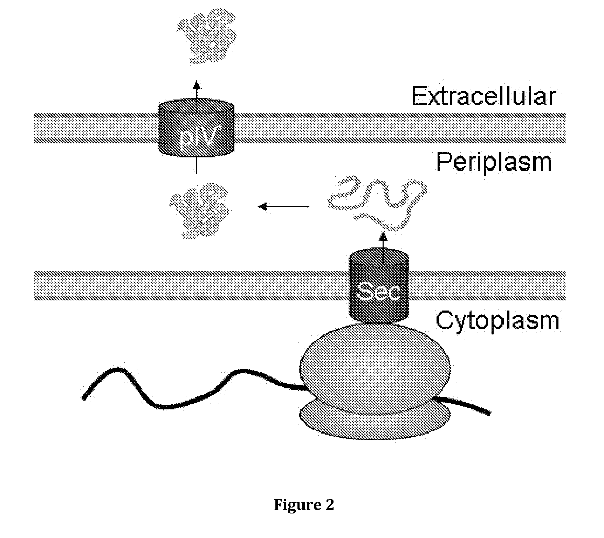

The general strategy for secretion of recombinant biomolecules in the extracellular medium is schematized in **Figure 2**.

### Cyclic peptide exportation to the extracellular medium.

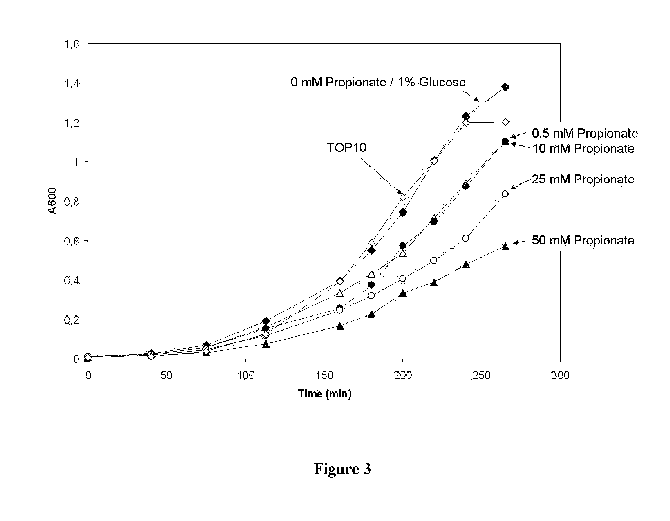

Ff bacteriophages such as F1, fd or M13 are filamentous particles of ~7 nm diameter that are using a self-encoded channel protein, pIV, to cross the outer membrane of *E.coli* without killing the bacterial cell. Marciano et al. (1999)¹ isolated a pIV mutant (pIV^{S324G}) from F1 allowing *E.coli* to grow on maltohexaose (MW: 991 g/mol) and rendering the bacteria sensitive to vancomycin, a large hydrophilic antibiotic (MW: 1449 g/mol) that is normally inactive towards *E.coli* because of its incapacity to pass through the bacterial outer membrane. Sequences of the pIV porines from F1, fd and M13 are more than 90% identical. Small cyclic peptides produced by the SICCLOPS method⁽²⁾ and exported to the periplasm⁽³⁾ were tested for their exportation into the extracellular medium by E. coli expressing the pIV^{S324G} gene from fd phage into the low copy pPRO30 vector⁽⁴⁾. A tunable propionate promoter controls pIV^{S324G'}s expression and allows setting the induction to a non toxic level. As shown in **Figure 3****,** the growth of E.coli (TOP10) transformed with the pPRO30-pIV^{S324G} vector is progressively affected by increasing concentrations of propionate. Under repression conditions (Glucose 1% w/v), the growth rate is similar to the untransformed bacteria. Without repression and at intermediate propionate concentration (up to 10 mM), the growth rate is moderately reduced whereas increased toxicity is observed at high propionate concentrations (25 and 50 mM). This confirms that the pPro30 vector is suitable for the expression of the pIV^{S324G} porine.

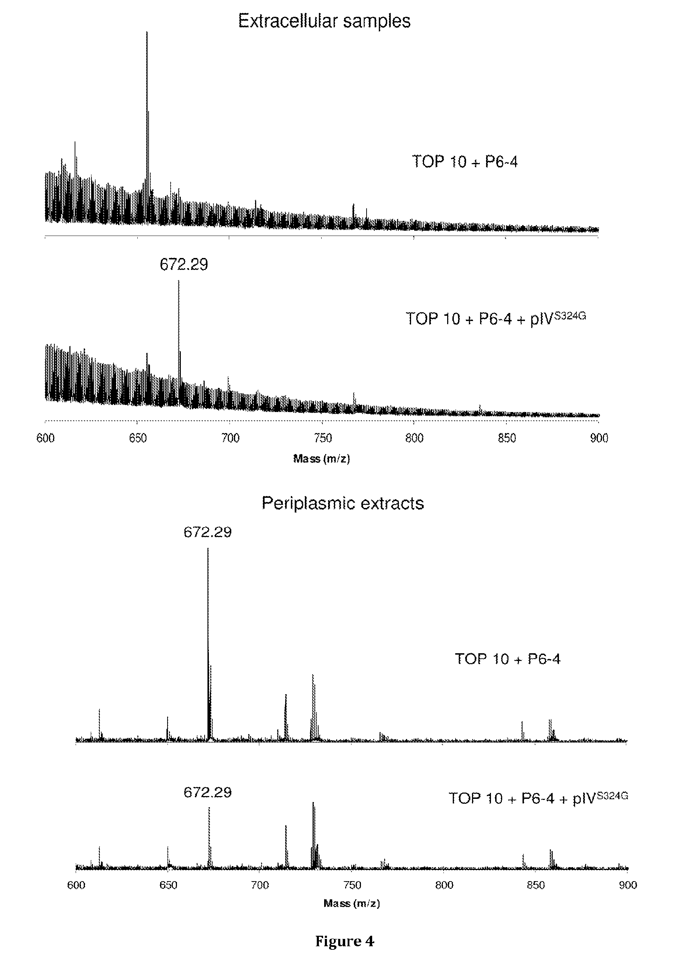

This vector is also compatible with the pBAD vector used for expressing the cyclic peptide. The peptide P6-4 used in this experiment is isolated from an expression library built as described in the protocols part. It is synthesized as a precursor comprising, from the amino-to the carboxy-terminus, the signal sequence of DsbA, the 48 residue C-terminal fragment of the Ssp DnaB intein, the sequence of the peptide and the 106 residue N-terminal fragment of the Ssp DnaB intein. After ribosomal synthesis and export into the periplasm, the signal sequence is cleaved and the intein splicing occurs, which results in the production of the cyclic peptide in the periplasm. Its sequence is Ser-Glu-Arg-Glu-Leu-Gly with a peptidic bond linking the amino- to the carboxy-terminus and its Mw is 672,32 g/mol. When we co-expressed the cyclic peptide with pIV^{S324G}, the peptide was easily detected in the extracellular medium while its MS signal was significantly decreased in the periplasmic extract **(****Figure 4****)**. In the absence of the pore, the peptide was barely detectable in the extracellular medium.

### Beta-lactamase exportation to the extracellular medium.

We showed that a small cyclic peptide could diffuse to the extracellular medium through the pIV^{S324G}. Considering the relatively large diameter of the pore (~6nm)⁽⁵⁾, we tested if small proteins such as tem-1 beta-lactamase (~30kDa) can diffuse as well through it. The pIV mutated gene was cloned into the low copy pPRO33 vector⁽⁴⁾ which is compatible with the pBAD vector used for expressing the tem-1 beta-lactamase to the periplasm. The pPRO33 vector is similar to the pPRO30 except that the ampicillin resistance gene is replaced by a chloramphenicol one.

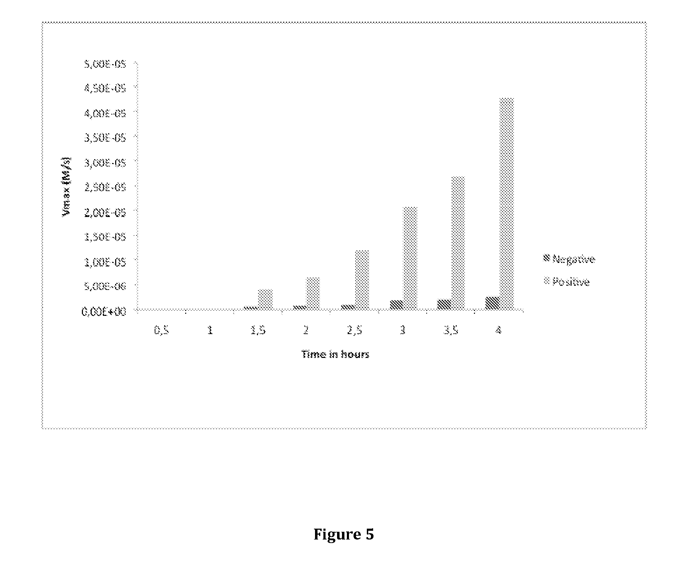

When we co-expressed the tem-1 beta-lactamase with pIV^{S324G}, the beta-lactamase activity was easily detected in the extracellular medium **(****Figure 5****).** After 4h, the beta-lactamase concentration in the extracellular medium was estimated to 6mg/L and has not reached a plateau. Moreover, this experiment was performed in an non-baffled Erlenmeyer so much higher yield should be obtained in fermentors. In the absence of the pore, the activity in the extracellular medium was much lower

### Bibliography:

1. Marciano, D.K., Russel, M. & Simon, S.M. An aqueous channel for filamentous phage export. *Science* **284**, 1516-1519 (1999).
2. Scott, C. P., Abel-Santos, E., Wall, M., Wahnon, D. C., and Benkovic, S. J. (1999). Production of cyclic peptides and proteins in vivo. *Proc Natl Acad Sci U S A. **96***, 13638-13643.
3. Deschuyteneer, G. & al. Efficient Intein-Mediated cyclization of randomized peptides in the periplasm of Escherichia coli and their extracellular secretion. Paper submitted in *ACS Chem. Biol.*
4. Lee, S.K. & Keasling, J.D. A Propionate-Inducible Expression System for Enteric Bacteria. *Appl Environ Microbiol.* **71**, 6856-6862 (2005).
5. Opalka, N. & al. Structure of the filamentous phage pIV multimer by cryo-electron microscopy. J. Mol. Biol 325, 461-470 (2003).
6. Soumillion, P. & Fastrez, J. A large decrease in heat-shock-induced proteolysis after tryptophan starvation leads to increased expression of phage lambda lysozyme cloned in Escherichia coli. Biochem. J 286 (Pt 1), 187-191(1992).

## Claims

1. A method for producing into the extracellular medium a biomolecule of interest, the method comprising the steps of:
(a) culturing a recombinant microorganism co-expressing:
- the mutant pIV^{S324G} porine into the outer membrane of the microorganism and
- a recombinant biomolecule of interest into the periplasm of the microorganism,
thereby secreting the biomolecule of interest into the extracellular medium; and
(b) recovering the biomolecule of interest from the extracellular medium.

2. The method according to claim **1,** comprising the steps of:
(a) culturing a recombinant microorganism co-transformed with:
- a first expression system allowing the production of the mutant pIV^{S324G} porine into the outer membrane of the microorganism and
- a second expression system allowing the production of the recombinant biomolecule of interest into the periplasm of the microorganism,
thereby secreting the protein of interest into the extracellular medium; and
(b) recovering the recombinant biomolecule of interest from the extracellular medium.

3. The method according to anyone of claim **1** to **2,** wherein the recombinant microorganism is a gram-negative bacterium.

4. The method according to claim **3,** wherein the gram-negative bacterium is Escherichia coli.

5. The method according to anyone of claim **1** to **4,** wherein the first expression system comprises a low copy plasmid.

6. The method according to claim **5,** wherein the low copy plasmid is the pPRO30 or pPRO33 comprising the PprpB promoter or the lac promoter.

7. The method according to anyone of claim **1** to **6,** wherein the second expression system comprises a high copy promoter.

8. The method according to claim **7,** wherein the high copy promoter is the T7 promoter or the P_{BAD} promoter.

9. The method according to anyone of claim **1** to **8,** wherein the second expression system comprises a signal sequence for addressing the protein of interest to the periplasm.

10. The method according to claim **9,** wherein the signal sequence is the sequence from the DsbA protein of E.coli.

11. The method according to anyone of the claims **1** to **10,** wherein at least 0,1 g/l of the protein of interest is recovered.


SEQUENCE LISTING

<110>  Université Catholique de Louvain
       Soumillion, Patrice

<120>  METHOD FOR EXTRACELLULAR PRODUCTION OF RECOMBINANT BIOMOLECULES
       IN A MICROORGANISM

<130>  Soumillion

<160>  17

<170>  PatentIn version 3.3

<210>  1
<211>  1281
<212>  DNA
<213>  artificial

<220>
<223>  mutant pIVS342G porine

<400>  1
atgaaattgt taaatgtaat taattttgtt ttcttgatgt ttgtttcatc atcttctttt      60

gctcaagtaa ttgaaatgaa taattcgcct ctgcgcgatt tcgtgacttg gtattcaaag     120

caaacaggtg aatctgttat tgtctcacct gatgttaaag gtacagtgac tgtatattcc     180

tctgacgtta agcctgaaaa tttacgcaat ttctttatct ctgttttacg tgctaataat     240

tttgatatgg ttggctctaa tccttccata attcagaaat ataacccaaa tagtcaggat     300

tatattgatg aattgccatc atctgatatt caggaatatg atgataattc cgctccttct     360

ggtggtttct ttgttccgca aaatgataat gttactcaaa catttaaaat taataacgtt     420

cgcgcaaagg atttaataag ggttgtagaa ttgtttgtta aatctaatac atctaaatcc     480

tcaaatgtat tatctgttga tggttctaac ttattagtag ttagcgcccc taaagatatt     540

ttagataacc ttccgcaatt tctttctact gttgatttgc caactgacca gatattgatt     600

gaaggattaa ttttcgaggt tcagcaaggt gatgctttag atttttcctt tgctgctggc     660

tctcagcgcg gcactgttgc tggtggtgtt aatactgacc gtctaacctc tgttttatct     720

tctgcgggtg gttcgttcgg tatttttaac ggcgatgttt tagggctatc agttcgcgca     780

ttaaagacta atagccattc aaaaatattg tctgtgcctc gtattcttac gctttcaggt     840

cagaagggtt ctatttctgt tggccagaat gtccctttta ttactggtcg tgtaactggt     900

gaatctgcca atgtaaataa tccatttcag acaattgagc gtcaaaatgt tggtatttct     960

atgagtgttt ttcccgttgc aatggctggc ggtaatattg ttttagatat aaccagtaag    1020

gccgatagtt tgggttcttc tactcaggca agtgatgtta ttactaatca aagaagtatt    1080

gcgacaacgg ttaatttgcg tgatggtcag actcttttgc tcggtggcct cactgattac    1140

aaaaacactt ctcaagattc tggtgtgccg ttcctgtcta aaatcccttt aatcggcctc    1200

ctgtttagct cccgttctga ttctaacgag gaaagcacgt tgtacgtgct cgtcaaagca    1260

accatagtac gcgccctgta g                                              1281

<210>  2
<211>  426
<212>  PRT
<213>  artificial

<220>
<223>  mutant pIVS342G porine

<400>  2

Met Lys Leu Leu Asn Val Ile Asn Phe Val Phe Leu Met Phe Val Ser
1               5                   10                  15

Ser Ser Ser Phe Ala Gln Val Ile Glu Met Asn Asn Ser Pro Leu Arg
            20                  25                  30

Asp Phe Val Thr Trp Tyr Ser Lys Gln Thr Gly Glu Ser Val Ile Val
        35                  40                  45

Ser Pro Asp Val Lys Gly Thr Val Thr Val Tyr Ser Ser Asp Val Lys
    50                  55                  60

Pro Glu Asn Leu Arg Asn Phe Phe Ile Ser Val Leu Arg Ala Asn Asn
65                  70                  75                  80

Phe Asp Met Val Gly Ser Asn Pro Ser Ile Ile Gln Lys Tyr Asn Pro
                85                  90                  95

Asn Ser Gln Asp Tyr Ile Asp Glu Leu Pro Ser Ser Asp Ile Gln Glu
            100                 105                 110

Tyr Asp Asp Asn Ser Ala Pro Ser Gly Gly Phe Phe Val Pro Gln Asn
        115                 120                 125

Asp Asn Val Thr Gln Thr Phe Lys Ile Asn Asn Val Arg Ala Lys Asp
    130                 135                 140

Leu Ile Arg Val Val Glu Leu Phe Val Lys Ser Asn Thr Ser Lys Ser
145                 150                 155                 160

Ser Asn Val Leu Ser Val Asp Gly Ser Asn Leu Leu Val Val Ser Ala
                165                 170                 175

Pro Lys Asp Ile Leu Asp Asn Leu Pro Gln Phe Leu Ser Thr Val Asp
            180                 185                 190

Leu Pro Thr Asp Gln Ile Leu Ile Glu Gly Leu Ile Phe Glu Val Gln
        195                 200                 205

Gln Gly Asp Ala Leu Asp Phe Ser Phe Ala Ala Gly Ser Gln Arg Gly
    210                 215                 220

Thr Val Ala Gly Gly Val Asn Thr Asp Arg Leu Thr Ser Val Leu Ser
225                 230                 235                 240

Ser Ala Gly Gly Ser Phe Gly Ile Phe Asn Gly Asp Val Leu Gly Leu
                245                 250                 255

Ser Val Arg Ala Leu Lys Thr Asn Ser His Ser Lys Ile Leu Ser Val
            260                 265                 270

Pro Arg Ile Leu Thr Leu Ser Gly Gln Lys Gly Ser Ile Ser Val Gly
        275                 280                 285

Gln Asn Val Pro Phe Ile Thr Gly Arg Val Thr Gly Glu Ser Ala Asn
    290                 295                 300

Val Asn Asn Pro Phe Gln Thr Ile Glu Arg Gln Asn Val Gly Ile Ser
305                 310                 315                 320

Met Ser Val Phe Pro Val Ala Met Ala Gly Gly Asn Ile Val Leu Asp
                325                 330                 335

Ile Thr Ser Lys Ala Asp Ser Leu Gly Ser Ser Thr Gln Ala Ser Asp
            340                 345                 350

Val Ile Thr Asn Gln Arg Ser Ile Ala Thr Thr Val Asn Leu Arg Asp
        355                 360                 365

Gly Gln Thr Leu Leu Leu Gly Gly Leu Thr Asp Tyr Lys Asn Thr Ser
    370                 375                 380

Gln Asp Ser Gly Val Pro Phe Leu Ser Lys Ile Pro Leu Ile Gly Leu
385                 390                 395                 400

Leu Phe Ser Ser Arg Ser Asp Ser Asn Glu Glu Ser Thr Leu Tyr Val
                405                 410                 415

Leu Val Lys Ala Thr Ile Val Arg Ala Leu
            420                 425

<210>  3
<211>  19
<212>  PRT
<213>  Escherichia coli

<400>  3

Met Lys Lys Ile Trp Leu Ala Leu Ala Gly Leu Val Leu Ala Phe Ser
1               5                   10                  15

Ala Ser Ala

<210>  4
<211>  44
<212>  DNA

<213>  artificial

<220>
<223>  primer IN-fp

<400>  4
aacagtgggt cttcgtgaag acgcggatgc atcagtggag atag                        44


<210>  5
<211>  28
<212>  DNA
<213>  artificial

<220>
<223>  primer IN-rp

<400>  5
atctagacta taattgtaaa gaggagct                                          28


<210>  6
<211>  28
<212>  DNA
<213>  artificial

<220>
<223>  primer IC-fp

<400>  6
gcccatggga tcaccagaaa tagaaaag                                          28


<210>  7
<211>  45
<212>  DNA
<213>  artificial

<220>
<223>  primer IC-rp

<400>  7
gcatccgcgt cttcacgaag acccactgtt atggacaatg atgtc                       45


<210>  8
<211>  35
<212>  DNA
<213>  artificial

<220>
<223>  primer INL(4-6)-fp wherein nbb is (nbb)4-6


<220>
<221>  misc_feature
<222>  (17)..(18)
<223>  n is a, c, g, or t

<400>  8
gggggaagac tccagtnnbg gatgcatcag tggag                                  35


<210>  9
<211>  23
<212>  DNA
<213>  artificial

<220>
<223>  primer INL-rp

<400>  9
gccaagctgg agaccgttta act                                              23


<210>  10
<211>  21
<212>  DNA
<213>  artificial

<220>
<223>  primer ICL-fp

<400>  10
ccccgcttat taaaagcatt c                                                21


<210>  11
<211>  38
<212>  DNA
<213>  artificial

<220>
<223>  primer ICL-rp

<400>  11
cagcattacc cagtcaccat gaagacccac tgttatgg                              38


<210>  12
<211>  56
<212>  DNA
<213>  artificial

<220>
<223>  primer pIV-fp

<400>  12
attcaactgc tagcaggagg aattccatat gaaattgtta aatgtaatta attttg          56


<210>  13
<211>  34
<212>  DNA
<213>  artificial

<220>
<223>  primer pIV-rp

<400>  13
ctaatcaagg atccctacag ggcgcgtact atgg                                  34


<210>  14
<211>  28
<212>  DNA
<213>  artificial

<220>
<223>  primer S324G-fp

<400>  14
ggccgatagt ttgggttctt ctactcag                                         28


<210>  15

<211>  28
<212>  DNA
<213>  artificial

<220>
<223>  primer S324G-rp

<400>  15
ctgagtagaa gaacccaaac tatcggcc                                          28


<210>  16
<211>  263
<212>  PRT
<213>  Escherichia coli

<400>  16

His Pro Glu Thr Leu Val Lys Val Lys Asp Ala Glu Asp Gln Leu Gly
1               5                   10                  15

Ala Arg Val Gly Tyr Ile Glu Leu Asp Leu Asn Ser Gly Lys Ile Leu
            20                  25                  30

Glu Ser Phe Arg Pro Glu Glu Arg Phe Pro Met Met Ser Thr Phe Lys
        35                  40                  45

Val Leu Leu Cys Gly Ala Val Leu Ser Arg Val Asp Ala Gly Gln Glu
    50                  55                  60

Gln Leu Gly Arg Arg Ile His Tyr Ser Gln Asn Asp Leu Val Glu Tyr
65                  70                  75                  80

Ser Pro Val Thr Glu Lys His Leu Thr Asp Gly Met Thr Val Arg Glu
                85                  90                  95

Leu Cys Ser Ala Ala Ile Thr Met Ser Asp Asn Thr Ala Ala Asn Leu
            100                 105                 110

Leu Leu Thr Thr Ile Gly Gly Pro Lys Glu Leu Thr Ala Phe Leu His
        115                 120                 125

Asn Met Gly Asp His Val Thr Arg Leu Asp Arg Trp Glu Pro Glu Leu
    130                 135                 140

Asn Glu Ala Ile Pro Asn Asp Glu Arg Asp Thr Thr Met Pro Ala Ala
145                 150                 155                 160

Met Ala Thr Thr Leu Arg Lys Leu Leu Thr Gly Glu Leu Leu Thr Leu
                165                 170                 175

Ala Ser Arg Gln Gln Leu Ile Asp Trp Met Glu Ala Asp Lys Val Ala
            180                 185                 190

Gly Pro Leu Leu Arg Ser Ala Leu Pro Ala Gly Trp Phe Ile Ala Asp
        195                 200                 205

Lys Ser Gly Ala Gly Glu Arg Gly Ser Arg Gly Ile Ile Ala Ala Leu
    210                 215                 220

Gly Pro Asp Gly Lys Pro Ser Arg Ile Val Val Ile Tyr Thr Thr Gly
225                 230                 235                 240

Ser Gln Ala Thr Met Asp Glu Arg Asn Arg Gln Ile Ala Glu Ile Gly
                245                 250                 255

Ala Ser Leu Ile Lys His Trp
            260

<210>  17
<211>  23
<212>  PRT
<213>  Escherichia coli

<400>  17

Met Ser Ile Gln His Phe Arg Val Ala Leu Ile Pro Phe Phe Ala Ala
1               5                   10                  15

Phe Cys Leu Pro Val Phe Ala
            20